# EUROPÄISCHE PATENTSCHRIFT

(11) **EP 1 999 099 B1**
(45) Veröffentlichungstag und Bekanntmachung des Hinweises auf die Patenterteilung: **15.02.2012**
(21) Anmeldenummer: 07726838.1
(22) Anmeldetag: 13.03.2007
(51) Int. Cl.: C07C 209/26, C07D 295/02

(54) **VERFAHREN ZUR HERSTELLUNG EINES AMINS**
PROCESS FOR PREPARING AN AMINE
PROCEDE DE FABRICATION D'UNE AMINE

(30) Priorität: 21.03.2006 EP 06111505
(43) Veröffentlichungstag der Anmeldung: 10.12.2008
(73) Patentinhaber: BASF SE, 67056 Ludwigshafen (DE)
(72) Erfinder: EBERHARDT, Jan, 68167 Mannheim (DE); HOFFER, Bram Willem, 69120 Heidelberg (DE); HAESE, Frank, 63128 Dietzenbach (DE); MELDER, Johann-Peter, 67459 Böhl-Iggelheim (DE); STEIN, Bernd, 64665 Alsbach-Hähnlein (DE); STANG, Michael, 67069 Ludwigshafen (DE); HILL, Thomas, 67071 Ludwigshafen (DE); SCHWAB, Ekkehard, 67434 Neustadt (DE)
(86) Internationale Anmeldenummer: PCT/EP2007/052332
(87) Internationale Veröffentlichungsnummer: WO 2007/107477

(56) Entgegenhaltungen:
- WO-A-02/074755
- DE-A1- 2 118 283
- DE-A1- 19 734 975

## Beschreibung

Die vorliegende Erfindung betrifft ein Verfahren zur Herstellung eines Amins durch Umsetzung eines Aldehyds und/oder Ketons mit Wasserstoff und einer Stickstoffverbindung, ausgewählt aus der Gruppe primärer und sekundärer Amine, in Gegenwart eines Heterogenkatalysators.

Die Verfahrensprodukte finden u.a. Verwendung als Zwischenprodukte bei der Herstellung von Kraftstoffadditiven (US-A-3,275,554; DE-A-21 25 039 und DE-A-36 11 230), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

Zur Herstellung eines Amins durch Umsetzung eines Aldehyds oder Ketons mit Wasserstoff und einer Stickstoffverbindung sind z.B. Hochdruckverfahren bekannt. Hier erfolgt die hydrierende Aminierung am Katalysatorfestbett, wobei z.B. Metallkatalysatoren mit Ni, Pd, Pt, Promotoren auf einem Träger eingesetzt werden.

DE-A-211 82 83 (BASF AG) betrifft ein Verfahren zur Herstellung sekundärer oder tertiärer aliphatischer oder cycloaliphatischer Amine unter Einsatz eines Pd/Ag-Katalysators, der keinen Schalenkatalysator darstellt. Trägermaterial ist insbesondere SiO₂.

EP-A1-7093 (BASF AG) betrifft die Herstellung von N-Aralkyl-2,6-dimethylmorpholinen, wie z.B. Fenpropimorph, an Pd/Ag-Katalysatoren, die keine Schalenkatalysatoren darstellen. Trägermaterial ist insbesondere SiO₂.

WO-A1-2002 074755 (= EP-A-1 373 232) (BASF AG) beschreibt die Herstellung von 2,6-Dialkylmorpholinen, wie z.B. Dodemorph, an Katalysatoren, deren aktive Komponente im wesentlichen aus Platingruppenmetallen besteht. Besonders bevorzugter Träger ist ZrO₂.

Die deutsche Patentanmeldung Nr. 102005019540.7 vom 27.04.05 (BASF AG) betrifft ein Verfahren zur Herstellung eines Amins durch Umsetzung eines Aldehyds und/oder Ketons mit Wasserstoff und einer Stickstoffverbindung, ausgewählt aus der Gruppe primärer und sekundärer Amine, in Gegenwart eines Heterogenkatalysators, wobei es sich bei dem Katalysator um eine Katalysatorpackung handelt, die herstellbar ist durch Aufbringen mindestens eines katalytisch aktiven Metalls und/oder mindestens einer Verbindung dieses Metalls auf ein Gewebe, ein Gestricke oder eine Folie als Trägermaterial.

Zur Herstellung eines Amins durch hydrierende Aminierung sind auch Niederdruckverfahren bekannt. Hier kommen z.B. Edelmetallkatalysatoren in Suspensionsfahrweise zum Einsatz, wie in US-A-4,521,624 (Ethyl Corp.) für die Herstellung von insbesondere N,N-Dimethylcyclohexylamin (DMCHA) an Pd/C beschrieben.

EP-A1-611 137 (Sumitomo Chem. Comp.) betrifft die reduktive Aminierung von zyklischen Ketonen; wobei in einer ersten Stufe eine entsprechende Imino-Verbindung hergestellt wird, die nachfolgend hydriert wird.

EP-A2-312 253 (Kao Corp.) beschreibt die Verwendung spezifischer Kupferkatalysatoren bei der Herstellung von n-substituierten Aminen aus Alkoholen oder Aldehyden.

Der vorliegenden Erfindung lag die Aufgabe zugrunde, unter Überwindung eines oder mehrerer Nachteile des Stands der Technik, ein verbessertes wirtschaftliches Verfahren zur Herstellung eines Amins aufzufinden. Insbesondere sollte das Verfahren einen Katalysator hoher Aktivität beinhalten, der eine besonders hohe Selektivität in der Reaktion zeigt.

Demgemäß wurde ein Verfahren zur Herstellung eines Amins durch Umsetzung eines Aldehyds und/oder Ketons mit Wasserstoff und einer Stickstoffverbindung, ausgewählt aus der Gruppe primärer und sekundärer Amine, in Gegenwart eines Heterogenkatalysators gefunden, welches dadurch gekennzeichnet ist, dass es sich bei dem Katalysator um einen Schalenkatalysator handelt, der mindestens ein Metall der VIII. Gruppe des Periodensystems der Elemente als Hydriermetall und zusätzlich einen Promotor auf einem oxidischen Träger umfasst, wobei mindestens 80 % des Metalls der VIII. Gruppe des Periodensystems der Elemente in einer Schicht zwischen der Oberfläche des Katalysators und einer Eindringtiefe, die maximal 80 % des Radius des Katalysators, gerechnet von der Oberfläche des Katalysators, entspricht, vorliegt, und der Promotor über den gesamten Querschnitt des katalysators im Wesentlichen homogen verteilt ist.

Bevorzugt liegt das Metall der VIII. Gruppe des Periodensystems der Elemente in der definierten Schale im wesentlichen homogen verteilt vor.

Zu den Vorteilen des erfindungsgemäßen Verfahrens zählt u. a. eine gute chemische Aktivität des Katalysators, die hohe mechanische Stabilität des Katalysatorträgers und die sehr gute Selektivität des Katalysators. Insbesondere wird die Überhydrierung des Einsatzmaterials (Keton oder Aldehyd) zum korrespondierenden Alkohol nur zu einem sehr geringen Maße beobachtet. In der Synthese resultieren folglich Einsatzstoffkosten-Vorteile. Mit dem erfindungsgemäßen Verfahren können auch Wirkstoffe mit definierter Stereochemie besonders vorteilhaft produziert werden, da die stereochemische

Information im Verlauf der Synthese mit hoher Selektivität erhalten bleibt. Auch werden bei der Synthese von unsymmetrisch-substituierten Aminen Nebenreaktionen wie die unselektive Übertragung von Substituenten nicht oder nur zu einem geringen Maße beobachtet. Die hohe Aktivität des erfindungsgemäß eingesetzten Katalysators ermöglicht es weiterhin, die Umsetzung bei verringertem Druck und/oder verringerter Temperatur durchzuführen, was die Selektivität der Reaktion zusätzlich erhöht. Die Möglichkeit, die reduktive Aminierung bei geringerem Druck wie z.B. 90 bar anstelle von 140 bar mit dennoch sehr hohen Raum-Zeit-Ausbeuten durchführen zu können, ermöglicht es, Produktionsanlagen mit deutlich geringeren Investitionskosten zu errichten (niedrigere Druckstufe).

Der im erfindungsgemäßen Verfahren eingesetzte Katalysator ist wie folgt charakterisiert und lässt sich wie folgt herstellen. Die Herstellung ist auch in der älteren BASF-Patentanmeldung PCT/EP2005/011026 vom 13.10.05 beschrieben (WO-A1-2006 040 159).

Der erfindungsgemäß eingesetzte Katalysator ist dadurch gekennzeichnet, dass mindestens 80 % des Metalls der VIII. Gruppe des Periodensystems der Elemente in einer Schicht zwischen der Oberfläche des Katalysators und einer Eindringtiefe, die maximal 80 % des Radius des Katalysators, gerechnet von der Oberfläche des Katalysators, entspricht, vorliegt, und der Promoter über den gesamten Querschnitt des katalysators im Wesentlichen homogen verteilt ist.

In einer bevorzugten Ausführungsform weist der Katalysator einen Durchmesser im Bereich von 1,5 bis 10 mm auf, wobei mindestens 80 % des Metalls der VIII. Gruppe des Periodensystems der Elemente in einer Schicht zwischen der Oberfläche des Katalysators und einer Eindringtiefe von maximal 1000 µm, gerechnet von der Oberfläche des Katalysators, vorliegt.

Bevorzugt liegt das Metall der VIII. Gruppe des Periodensystems der Elemente in der definierten Schale im wesentlichen homogen verteilt vor.

Erfindungsgemäß ist somit ein Katalysator vorgesehen, bei dem das Metall der VIII. Gruppe des Periodensystems der Elemente eine Schalenstruktur in dem Katalysator ausbildet.

Die Bezeichnung der Gruppen des Periodensystems der Elemente erfolgt gemäß der CAS-Nomenklatur (chemical abstracts service).

Der erfindungsgemäß eingesetzte Katalysator weist bevorzugt einen Durchmesser im Bereich von 1,5 bis 9 mm auf. In besonders bevorzugten Ausführungsformen beträgt der Durchmesser der erfindungsgemäß eingesetzten Katalysatoren 2,0 bis 5 mm, insbesondere 2,5 bis 3,5 mm.

In dem erfindungsgemäß eingesetzten Katalysator liegen bevorzugt mindestens 80 %, vorzugsweise mindestens 90 %, besonders bevorzugt mindestens 95 %, insbesondere mindestens 98 %, speziell 100 %, des Metalls der VIII. Gruppe des Periodensystems der Elemente in einer Schicht zwischen der Oberfläche des Katalysators und einer Eindringtiefe von maximal 1000 µm, gerechnet von der Oberfläche des Katalysators, vor.

Der erfindungsgemäß eingesetzte Katalysator enthält ein Metall der VIII. Gruppe des Periodensystems der Elemente (Fe, Ru, Os, Co, Rh, Ir, Ni, Pd, Pt). In einer bevorzugten Ausführungsform der vorliegenden Erfindung handelt es sich um Palladium.

Der erfindungsgemäß eingesetzte Katalysator enthält darüber hinaus mindestens einen Promotor. Beispielsweise kann es sich hierbei um weitere Metalle der VIII., IB. und IIB. Gruppe des Periodensystems der Elemente handeln (Cu, Ag, Au, Zn, Cd, Hg). In einer bevorzugten Ausführungsform enthalten die erfindungsgemäß eingesetzten Katalysatoren neben dem Metall der VIII. Gruppe des Periodensystems der Elemente noch mindestens ein Metall aus der IB. Gruppe des Periodensystems der Elemente. Besonders bevorzugt ist hierbei Silber.

In einer besonders bevorzugten Ausführungsform enthält der erfindungsgemäß eingesetzte Katalysator Palladium und Silber.

Der erfindungsgemäß eingesetzte Katalysator kann beliebige Formen, beispielsweise Stränge, Hohlstränge, Tabletten, Ringe, sphärische Teilchen oder Kugeln, aufweisen. Bevorzugt ist es, wenn der Katalysator als Strang ausgebildet ist.

Die Metalle können in reiner metallischer Form vorliegen, aber auch in Form von Verbindungen, beispielsweise in Form von Metalloxiden. Unter den Betriebsbedingungen des Aminierungsverfahrens liegen sie im Allgemeinen in Form von Metallen vor. Die Umwandlung etwaiger Oxide in Metalle kann auf dem Fachmann bekannte Weise vor dem Einsatz des Katalysators in einem Hydrierverfahren in oder außerhalb eines Hydrierreaktors, beispielsweise durch Vorreduzierung und, falls für Manipulationen mit dem vorreduzierten Katalysator erforderlich oder vorteilhaft, anschließende oberflächliche Passivierung erfolgen.

Der Gehalt des Katalysators an Metall oder Metallen der VIII. Gruppe des Periodensystems, insbesondere Palladium, beträgt vorzugsweise mindestens 0,01 Gew.-%, besonders bevorzugt mindestens 0,1 Gew.-%, insbesondere mindestens 0,15 Gew.-%. Vorzugsweise liegt dieser Gehalt bei höchstens 5 Gew.-%, besonders bevorzugt höchstens 1 Gew.-%, insbesondere höchstens 0,6 Gew.-%. Niedrigere und höhere Gehalte sind zwar möglich, aber im Normalfall wegen zu niedriger Aktivität oder zu hohen Rohstoffkosten wirtschaftlich unbefriedigend. In einer besonders bevorzugten Ausführungsform wird nur ein Hydriermetall, insbesondere Palladium, verwendet.

Das Verhältnis der Mengen von Hydriermetall der VIII. Gruppe des Periodensystems der Elemente und Zusatz- oder Dotierstoffen ist ein im Einzelfall zu optimierender Parameter. Vorzugsweise beträgt das Atomverhältnis von Metall der VIII. Gruppe des Periodensystems der Elemente, besonders bevorzugt Palladium, zu dem Promotor, besonders bevorzugt Silber, vorzugsweise 0,1 - 10, besonders bevorzugt 2 - 7, insbesondere 2,5 - 6.

Der oxidische Träger des erfindungsgemäß eingesetzten Katalysators ist bevorzugt Aluminiumoxid, besonders bevorzugt in einer Mischung aus δ-, θ- und α-Aluminiumoxid. Der Träger kann neben unvermeidbaren Verunreinigungen in gewissem Umfang auch andere Zusätze enthalten. Beispielsweise können andere anorganische Oxide wie Oxide von Metallen der IIA., IIIB., IVB., IIIA. und IVA. Gruppe des Periodensystems der Elemente enthalten sein, insbesondere Siliziumdioxid, Titandioxid, Zirkondioxid, Zinkoxid, Magnesiumoxid, Natriumoxid und/oder Kalziumoxid. Der maximale Gehalt des Trägers an solchen von Aluminiumoxid verschiedenen Oxiden ist vom tatsächlich vorhandenen Oxid abhängig, aber im Einzelfall anhand des Röntgenbeugungsdiagramms des Hydrierkatalysators zu ermitteln, da eine Änderung der Struktur mit einer signifikanten Änderung des Röntgenbeugungsdiagramms einher geht. Im Allgemeinen liegt der Gehalt an solchen, von Aluminiumoxid verschiedenen Oxiden, unterhalb von 50 Gew.-%, vorzugsweise unterhalb von 30 Gew.-%, besonders bevorzugt unterhalb von 10 Gew.-%. Der Reinheitsgrad des Aluminiumoxids liegt vorzugsweise höher als 99%.

Zur Herstellung des Trägers wird ein geeigneter aluminiumhaltiger Rohstoff, vorzugsweise Böhmit, mit einem Peptisierungsmittel, wie Wasser, verdünnter Säure oder verdünnter Base, peptisiert. Als Säure wird beispielsweise eine Mineralsäure, wie etwa Salpetersäure, oder eine organische Säure, wie etwa Ameisensäure, verwendet. Als Base wird vorzugsweise eine anorganische Base, wie etwa Ammoniak, verwendet. Die Säure oder Base wird im Allgemeinen in Wasser gelöst. Vorzugsweise werden als Peptisierungsmittel Wasser oder verdünnte wässerige Salpetersäure verwendet. Die Konzentration des nicht-wässrigen Anteils im Peptisierungsmittel beträgt im Allgemeinen 0 - 10 Gew.-%, vorzugsweise 0 - 7 Gew.-%, besonders bevorzugt 0 - 5 Gew.-%. Im Anschluss an die Peptisierung wird der Träger verformt, getrocknet und kalziniert.

Böhmit (γ-AlO(OH)) ist ein verbreitetes Handelsprodukt, kann aber auch in bekannter Weise unmittelbar vor der eigentlichen Trägerherstellung durch Fällung aus einer Lösung eines Aluminiumsalzes, beispielsweise Aluminiumnitrat, mit einer Base, Abtrennen, Waschen, Trocknen und Kalzinieren des gefällten Feststoffes hergestellt werden. Vorteilhafterweise wird Böhmit in der Form eines Pulvers verwendet. Ein geeignetes handelsübliches Böhmit-Pulver ist beispielsweise Versal^{®} 250, das von UOP erhältlich ist. Der Böhmit wird mit dem Peptisierungsmittel behandelt, indem er mit dem Peptisierungsmittel angefeuchtet und intensiv durchmischt wird, beispielsweise in einem Kneter, Mischer oder Kollergang. Die Peptisierung wird fortgesetzt, bis die Masse gut verformbar ist. Anschließend wird die Masse mittels üblicher Methoden zu den gewünschten Trägerformkörpern verformt, beispielsweise durch Strangpressen, Extrudieren, Tablettieren oder Agglomerieren. Zur Verformung ist jede bekannte Methode geeignet. Falls erforderlich oder vorteilhaft können übliche Zusätze verwendet werden. Beispiele für solche Zusätze sind Extrudier- oder Tablettierhilfsmittel, wie Polyglykole oder Graphit.

Es ist ferner möglich, der Trägerrohmasse vor der Verformung Zusätze beizumischen, die in bekannter Weise als Ausbrennstoffe die Porenstruktur des Trägers nach der Kalzination beeinflussen, beispielsweise Polymere, Faserstoffe, natürliche Ausbrennstoffe, wie Nussschalenmehle, oder andere übliche Zusätze. Bevorzugt ist die Verwendung von Böhmit in einer Korngrößenverteilung und die Zugabe von Ausbrennstoffen, die zu einer Porenradienverteilung des fertigen Trägers führt, bei der 50 - 90 Vol.-% des gesamten Porenvolumens in Form von Poren mit einem mittleren Durchmesser im Bereich von 0,01 - 0,1 µm und 10 - 50 Vol.-% des gesamten Porenvolumens in Form von Poren mit einem mittleren Durchmesser im Bereich von 0,1 - 1 µm vorliegen. Die hierzu notwendigen Maßnahmen sind dem Fachmann an sich bekannt.

Im Anschluss an die Verformung werden die Formkörper in üblicher Weise getrocknet, im Allgemeinen bei einer Temperatur oberhalb von 60°C, vorzugsweise oberhalb von 80°C, besonders bevorzugt oberhalb von 100°C, insbesondere bei einer Temperatur im Bereich von 120 - 300°C. Die Trocknung wird fortgesetzt, bis in Formkörpern vorhandenes Wasser im wesentlichen vollständig aus den Formkörpern entwichen ist, was im Allgemeinen nach einigen Stunden der Fall ist. Übliche Trocknungsdauern liegen im Bereich von 1 bis 30 Stunden und sind von der eingestellten Trocknungstemperatur abhängig, wobei eine höhere Temperatur die Trocknungszeit verkürzt. Die Trocknung kann durch Anwenden eines Unterdrucks weiter beschleunigt werden.

Im Anschluss an die Trocknung werden die Formkörper durch Kalzination zum fertigen Träger umgewandelt. Die Kalzinationstemperatur liegt im Allgemeinen im Bereich von 900 - 1150°C, vorzugsweise im Bereich von 1000 - 1120°C, besonders bevorzugt im Bereich von 1050 - 1100°C. Die Kalzinationsdauer liegt im Allgemeinen zwischen 0,5 und 5 Stunden, vorzugsweise zwischen 1 und 4 Stunden, besonders bevorzugt zwischen 1,5 und 3 Stunden. Die Kalzination erfolgt in einem üblichen Ofen, beispielsweise in einem Drehofen, in einem Tunnelofen, in einem Bandkalzinierer oder in einem Kammerofen. Die Kalzination kann sich ohne zwischenzeitliche Abkühlung der Formkörper direkt an die Trocknung anschließen.

Die so erhaltenen erfindungsgemäß einsetzbaren Katalysatoren weisen eine spezifische Oberfläche (BET, Brunauer - Emmet - Teller, bestimmt gemäß DIN 66131 durch Stickstoffadsorption bei 77 K) von 20 - 250 m²/g, vorzugsweise 50 - 150 m²/g, insbesondere 60 - 90 m²/g, auf. Die Oberfläche kann durch bekannte Methoden, insbesondere Verwendung feinteiliger oder gröberer Ausgangsstoffe, Kalzinationsdauer und Kalzinationstemperatur, variiert werden. Wie die BET-Oberfläche kann auch das Porenvolumen auf bekannte Weise variiert werden, im Allgemeinen liegt es, mittels Quecksilberporosymmetrie bestimmt, in einem Bereich von 0,3 - 1,0 ml/g, vorzugsweise in einem Bereich von 0,4 - 0,9 ml/g, besonders bevorzugt 0,5 - 0,8 ml/g.

Nach der Kalzination werden auf dem so hergestellten Träger die Aktivmasse und gegebenenfalls weitere Zusatzstoffe abgeschieden.

Der Träger des erfindungsgemäßen Katalysators ist vorzugsweise durch folgendes Röntgenbeugungsdiagramm charakterisiert:

| Netzebenenabstand | Winkel | relative Intensität |
|---|---|---|
| Angström [Å] | 2-Theta [°] | [%] |
| | | |
| d = 4,552 | 19,483 | 5 -15 |
| d = 2,857 | 31,278 | 35 - 50 |
| d = 2,730 | 32,775 | 65 - 80 |
| d = 2,449 | 36,671 | 45 - 55 |
| d = 2,317 | 38,842 | 35 - 45 |
| d = 2,260 | 39,861 | 5 - 45 |
| d = 2,022 | 44,790 | 45 - 65 |
| d = 1,910 | 47,570 | 30 - 40 |
| d = 1,798 | 50,720 | 10 - 25 |
| d = 1,543 | 59,915 | 25-35 |
| d = 1,511 | 61,307 | 0 - 35 |
| d = 1,489 | 62,289 | 20 - 30 |
| d = 1,455 | 63,926 | 25 - 35 |
| d = 1,387 | 67,446 | 100 |

Dieses Röntgenbeugungsdiagramm wird wie in der EP 0 992 284 A2 auf Seite 9, Zeilen 6 bis 9 beschrieben, bestimmt.

Röntgenbeugungsdiagramme sind charakteristisch für die spezifische Struktur des untersuchten Materials. Die Struktur des erfindungsgemäßen Katalysators ist durch das Auftreten der oben genannten Reflexe hinreichend definiert. Zusätzlich zu den oben angegebenen kennzeichnenden Reflexen können im Röntgenbeugungsdiagramm ein oder mehrere Reflexe in beliebiger Intensität für die Netzebenenabstände 3,48; 2,55; 2,38; 2,09; 1,78; 1,74; 1,62; 1,60; 1,57; 1,42; 1,40 und/oder 1,37 alle in der Einheit [Ä], auftreten.

Weiterhin können im Röntgenbeugungsdiagramm des erfindungsgemäß eingesetzten Katalysators noch beliebige weitere Reflexe auftreten.

Auf den so erhaltenen Träger des erfindungsgemäß eingesetzten Katalysators können die Aktivmasse und gegebenenfalls weitere Zusatzstoffe abgeschieden werden.

Die auf den Träger abzuscheidenden Metalle, Zusatz- und/oder Dotierstoffe (= Promotoren) können mit jedem bekannten Verfahren auf den Träger aufgebracht werden, beispielsweise durch Beschichtung aus der Gasphase (chemical oder physical vapor deposition) oder Tränkung des Trägermaterials in einer Lösung, welche die abzuscheidenen Substanzen und/oder Verbindungen enthält.

Die bevorzugte Methode ist die Tränkung mit einer Lösung der abzuscheidenden Substanzen und/oder Verbindungen, die sich im Zuge der weiteren Katalysatorherstellung in die abzuscheidenden Substanzen umwandeln. Die abzuscheidenden Substanzen können einzeln und/oder in Teilmengen in mehreren Verfahrensschritten oder gemeinsam und vollständig in einem Verfahrensschritt abgeschieden werden. Bevorzugt ist die gemeinsame Abscheidung in einer Tränkstufe. Im Anschluss an die Tränkung oder nach den einzelnen Tränkstufen wird der geträgerte Katalysator getrocknet und durch Kalzinierung sowie gegebenenfalls andere bekannte Nachbehandlungsmethoden, beispielsweise Aktivierung und anschließende oberflächliche Passivierung, zum einsatzbereiten Katalysator umgewandelt.

Tränkverfahren zur Abscheidung von Aktivkomponenten, Zusatzstoffen und/oder Dotierstoffen auf einem Träger sind bekannt. Im Allgemeinen wird der Träger mit einer Lösung von Salzen der abzuscheidenden Komponenten getränkt, wobei das Volumen der Lösung so bemessen wird, dass die Lösung praktisch vollständig vom Porenvolumen des Trägers aufgenommen wird ("incipient wetness"-Methode). Die Konzentration der Salze in der Lösung wird so bemessen, dass nach Tränkung und Umwandlung des geträgerten Katalysators zum fertigen Katalysator die abzuscheidenden Komponenten in der gewünschten Konzentration auf dem Katalysator vorliegen. Die Salze werden so gewählt, dass sie keine bei der Katalysatorherstellung oder dessen späterer Verwendung störenden Rückstände hinterlassen. Zumeist werden Nitrate oder Ammoniumsalze verwendet.

Grundsätzlich eignen sich alle dem Fachmann bekannten Tränkverfahren zur Herstellung des erfindungsgemäß eingesetzten Katalysators.

Die Herstellung des erfindungsgemäß eingesetzten Katalysators erfolgt jedoch vorzugsweise unter einstufiger Tränkung des Trägers nach der incipient-wetness-Methode mit einer salpetersauren Lösung der Nitrate der abzuscheidenden Metalle.

In einer besonders bevorzugten Ausführungsform wird eine Tränklösung verwendet, die Palladiumnitrat und -nitrit nebeneinander enthält.

Darüber hinaus liegt in der Tränklösung bevorzugt noch das Metall der IB. Gruppe des Periodensystems der Elemente, vorzugsweise Silbernitrat, vor.

Im Allgemeinen liegt der pH-Wert der Tränklösung bei höchstens 5, vorzugsweise bei höchstens 2, besonders bevorzugt bei höchstens 1, insbesondere bei höchstens 0,5. Die Untergrenze des pH-Wertes liegt im Allgemeinen bei 0,2, vorzugsweise bei 0,3, besonders bevorzugt bei 0,5. Ein bevorzugter pH-Bereich liegt z.B. bei 0,2 bis 2, insbesondere bei 0,3 bis 0,5.

Nach der Tränkung wird der getränkte Träger in üblicher Weise getrocknet, im Allgemeinen bei einer Temperatur oberhalb von 60°C, vorzugsweise oberhalb von 80°C, besonders bevorzugt oberhalb von 100°C, insbesondere bei einer Temperatur im Bereich von 120 - 300°C. Die Trocknung wird dabei fortgesetzt, bis im getränkten Katalysator vorhandenes Wasser im wesentlichen vollständig entwichen ist, was im Allgemeinen nach einigen Stunden der Fall ist. Übliche Trocknungszeiten liegen im Bereich von 1 - 30 Stunden und sind von der eingestellten Trocknungstemperatur abhängig, wobei eine höhere Trocknungstemperatur die Trocknungszeit verkürzt. Die Trocknung kann durch Anwenden eines Unterdrucks weiter beschleunigt werden.

In einer besonders bevorzugten Ausführungsform zur Herstellung des erfindungsgemäß eingesetzten Katalysators erfolgt die Trocknung des getränkten Katalysators unter gleichzeitiger Bewegung des getränkten Trägermaterials, beispielsweise in einem Drehrohr-Ofen.

In einer besonderen Ausführungsform wird der zur Trocknung verwendete Luftstrom im Gegenstrom durch das Drehrohr geführt.

Im Anschluss an die Trocknung wird in üblicher Weise durch Kalzinierung der Katalysator hergestellt. Diese Kalzinierung dient im wesentlichen der Umwandlung der aufgetränkten Salze in die abzuscheidenden Komponenten oder Vorläufer solcher Komponenten und unterscheidet sich insofern von der zuvor beschriebenen Kalzinierung, die der Herstellung des Trägermaterials und der Trägerstruktur dient. Im Fall der Auftränkung von Metallnitraten werden bei dieser Kalzinierung im wesentlichen die Nitrate in Metalle und/oder Metalloxide, die im Katalysator verbleiben, und in nitrose Gase, die entweichen, zersetzt.

Die Kalzinationstemperatur liegt im Allgemeinen bei 200 - 900°C, vorzugsweise 280 - 800°C, besonders bevorzugt 300 - 700°C. Die Kalzinationsdauer liegt im Allgemeinen zwischen 0,5 und 20 Stunden, vorzugsweise zwischen 0,5 und 10 Stunden, besonders bevorzugt zwischen 0,5 und 5 Stunden. Die Kalzination erfolgt in einem üblichen Ofen, beispielsweise in einem Drehrohr-Ofen, in einem Bandkalzinierer oder in einem Kammerofen. Die Kalzination kann sich ohne zwischenzeitliche Abkühlung des geträgerten und getrockneten Katalysators direkt an die Trocknung anschließen.

In einer besonders bevorzugten Ausführungsform erfolgt die Trocknung und die Kalzinierung des Katalysators in einem Drehrohrofen kombiniert.

Um die Konzentrationen von Metall der VIII. Gruppe, z.B. Palladium, und Promotor, z.B. Silber, über der Strangdurchschnitt zu bestimmen, können dem Fachmann bekannte Methoden angewendet werden. Einer dieser Methoden ist ElektronenMikroskopie, z.B. Scanning Electron Microscopy (SEM) oder Electron Probe Micro Analysis (EPMA). Eine andere Technik ist, den Katalysatorstrang durch zu schneiden, zu behandeln mit einem reduzierenden Mittel (z.B. Wasserstoff), um eine Farbveränderung zu bewirken, um so die Verteilung der Metalle festzustellen.

Nach der Kalzination ist der Katalysator prinzipiell einsatzbereit. Falls erforderlich oder gewünscht, wird er vor dem Einbau in den Reaktor der aminierenden Hydrierung in bekannter Weise durch Vorreduzierung aktiviert und gegebenenfalls auch wieder oberflächlich passiviert.

In der Regel erfolgt die Reduktion des Katalysators jedoch meistens erst im Reaktor der aminierenden Hydrierung selbst. Dies geschieht nach einer dem Fachmann bekannten Weise durch zunächst erfolgende Inertisierung mit Stickstoff oder einem anderen Inertgas. Die Reduktion wird, mit einem Wasserstoff-haltigen Gas als reine Gasphase oder unter Inert-Kreislauf durchgeführt. Die Temperatur, bei der diese Vorreduktion durchgeführt wird, beträgt im Allgemeinen 5 - 200°C, vorzugsweise 20 - 150°C.

Auch eine Regenerierung des erfindungsgemäß eingesetzten Katalysators ist außeroder innerhalb des Reaktors der aminierenden Hydrierung bei Temperaturen von 15 bis 500°C möglich.

Die vorstehend beschriebenen Katalysatoren werden erfindungsgemäß eingesetzt in einem Verfahren zur Herstellung eines Amins durch Umsetzung eines Aldehyds und/oder Ketons mit Wasserstoff und einer Stickstoffverbindung, ausgewählt aus der Gruppe primärer und sekundärer Amine (aminierende Hydrierung).

Durch den Einsatz der Katalysatoren können diese Aldehyde und Ketone mit hoher Selektivität und hoher Ausbeute in die entsprechenden sekundären und tertiären Amine überführt werden.

Die Aminierung der Carbonylverbindung erfolgt bevorzugt in der Flüssigphase.

Bei der Aminierung in Flüssigphase kann ein adiabatisch betriebener Reaktor mit oder ohne Rückführung genügen.

Der Katalysator ist im Reaktor der aminierenden Hydrierung (z.B. Rohrreaktor) bevorzugt als Festbett angeordnet.

Gemäß einer Ausführungsform der Erfindung wird die Umsetzung in der Flüssigphase oder in einer gemischten Flüssig-/Gasphase mit mindestens 50 Gew.-% des Reaktionsgemisches in der Flüssigphase durchgeführt.

Dabei kann die Aminierung gemäß einer Ausführungsform der Erfindung in der Rieselfahrweise oder in der Sumpffahrweise durchgeführt werden.

Bei der Sumpffahrweise kann der zugegebene Hydrierwasserstoff in der Flüssigphase gelöst vorliegen.

Die Eintrittstemperatur des Eduktgemisches bei der Aminierung beträgt gemäß einer Ausführungsform der Erfindung -10 bis 250°C, vorzugsweise 0 bis 180°C, insbesondere 50 bis 150°C.

Um die Ausbildung einer Flüssigphase zu gewährleisten, müssen geeignete Temperatur- und Druckparameter in den o.g. Bereichen gewählt werden, was abhängig vom jeweilig eingesetzten Stoffgemisch ist.

Die Stickstoffverbindung wird bevorzugt in der 0,90- bis 100-fachen molaren Menge, insbesondere in der 1,0- bis 10-fachen molaren Menge, jeweils bezogen auf den/das eingesetzte/n Aldehyd und/oder Keton eingesetzt.

Das erfindungsgemäße Verfahren wird bevorzugt bei einer Katalysatorbelastung - gemessen als Masse an Aldehyd oder Keton im Zulauf bezogen auf das Katalysatorvolumen und die Zeit - im Bereich von 0,01 bis 2,00 kg (Carbonylverbindung)/Liter (Katalysator)/h, bevorzugt 0,10 bis 1,50 kg/Liter/h, weiter bevorzugt 0,20 bis 1,20 kg/Liter/h, besonders bevorzugt 0,22 bis 1,00 kg/Liter/h, durchgeführt.

Das erfindungsgemäße Verfahren wird bevorzugt bei einem Absolutdruck im Bereich von 1 bis 325 bar, bevorzugt 10 bis 250 bar, weiter bevorzugt 100 bis 200 bar, besonders bevorzugt 85 bis 150 bar, z.B. 90 bis 135 bar, durchgeführt.

Das erfindungsgemäße Verfahren der Aldehyd- und/oder Ketonaminierung wird bevorzugt bei einer Temperatur im Bereich von 50 bis 280°C, bevorzugt 80 bis 250°C, besonders bevorzugt 120 bis 210°C, durchgeführt.

Bevorzugt wird eine Abgasmenge von 5 bis 800 Normkubikmeter/h, insbesondere 20 bis 300 Normkubikmeter/h, gefahren.

Die Anwendung höherer Temperaturen, höherer Gesamtdrücke und höherer Lasten ist möglich. Der Druck im Reaktor, welcher sich aus der Summe der Partialdrücke des Aminierungsmittels, der Aldehyd- und/oder Keton-Komponente und der gebildeten Reaktionsprodukte bei den angegebenen Temperaturen ergibt, wird zweckmäßigerweise durch Aufpressen von Wasserstoff auf den gewünschten Reaktionsdruck erhöht.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z.B. destillativ.

Aus dem Reaktionsaustrag werden, nachdem dieser zweckmäßigerweise entspannt worden ist, der überschüssige Wasserstoff und das gegebenenfalls vorhandene überschüssige Aminierungsmittel entfernt und das erhaltene Reaktionsrohprodukt gereinigt, z.B. durch eine fraktionierende Rektifikation. Geeignete Aufarbeitungsverfahren sind z.B. in EP-A-1 312 600 und EP-A-1 312 599 (beide BASF AG) beschrieben.

Unumgesetzte Edukte und gegebenenfalls anfallende geeignete Nebenprodukte können wieder in die Synthese zurückgeführt werden. Nicht umgesetzte Edukte können in diskontinuierlicher oder kontinuierlicher Fahrweise nach Kondensation der Produkte im Abscheider in dem Kreisgasstrom erneut über das Katalysatorbett geströmt werden.

Mit dem erfindungsgemäßen Verfahren herstellbar sind z.B. Amine der Formel I 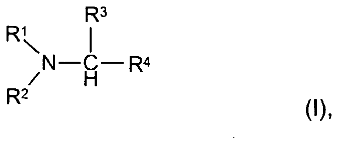 in der
- R¹, R²: Wasserstoff (H), Alkyl, wie C₁₋₂₀Alkyl, Cycloalkyl, wie C₃₋₁₂-Cycloalkyl, Alkoxyalkyl, wie C₂₋₃₀-Alkoxyalkyl, Dialkylaminoalkyl, wie C₃₋₃₀-Dialkylaminoalkyl, Aryl, Aralkyl, wie C₇₋₂₀-Aralkyl, und Alkylaryl, wie C₇₋₂₀-Alkylaryl, oder gemeinsam -(CH₂)ⱼ-X-(CH₂)ₖ-,
- R³, R⁴: Wasserstoff (H), Alkyl, wie C₁₋₂₀-Alkyl, Cycloalkyl, wie C₃₋₁₂-Cycloalkyl, Hydroxyalkyl, wie C₁₋₂₀-Hydroxyalkyl, Aminoalkyl, wie C₁₋₂₀-Aminoalkyl, Hydroxyalkylaminoalkyl, wie C₂₋₂₀- Hydroxyalkylaminoalkyl, Alkoxyalkyl, wie C₂₋₃₀-Alkoxyalkyl, Dialkylaminoalkyl, wie C₃₋₃₀-Dialkylaminoalkyl, Alkylaminoalkyl, wie C₂₋₃₀-Alkylaminoalkyl, R⁵-(OCR⁶R⁷CR⁸R⁹)ₙ(OCR⁶R⁷), Aryl, Heteroaryl, Aralkyl, wie C₇₋₂₀-Aralkyl, Heteroarylalkyl, wie C₄₋₂₀-Heteroarylalkyl, Alkylaryl, wie C₇₋₂₀-Alkylaryl, Alkylheteroaryl, wie C₄₋₂₀-Alkylheteroaryl, und Y-(CH₂)ₘ-NR⁵-(CH₂)_{q} oder gemeinsam -(CH₂)ₗX-(CH₂)ₘ- oder
- R² und R⁴: gemeinsam -(CH₂)ₗ-X-(CH₂)ₘ-,
- R⁵, R¹⁰: Wasserstoff (H), Alkyl, wie C₁₋₄-Alkyl, Alkylphenyl, wie C₇₋₄₀-Alkylphenyl,
- R6, R⁷, R⁸, R⁹: Wasserstoff (H), Methyl oder Ethyl,
- X: CH₂, CHR⁵, Sauerstoff (O), Schwefel (S) oder NR⁵,
- Y: N(R¹⁰)₂, Hydroxy, C₂₋₂₀-Alkylaminoalkyl oder C₃₋₂₀-Dialkylaminoalkyl,
- n: eine ganze Zahl von 1 bis 30 und
- j, k, l, m, q: eine ganze Zahl von 1 bis 4,
bedeuten.

Das erfindungsgemäße Verfahren findet daher bevorzugt zur Herstellung eines Amins I Anwendung, indem man einen Aldehyd und/oder ein Keton der Formel VI bzw. VII 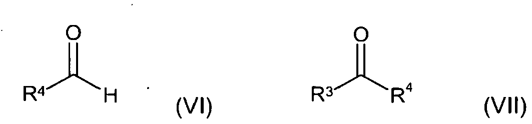 mit einer Stickstoffverbindung der Formel III 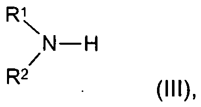 wobei R¹, R², R³ und R⁴ die oben genannten Bedeutungen haben, umsetzt.

Wie aus den Definitionen für die Reste R² und R⁴ hervorgeht, kann die Umsetzung auch intramolekular in einem entsprechenden Aminoketon oder Aminoaldehyd erfolgen.

Zur Herstellung des Amins I wird demnach rein formal ein Wasserstoffatom der Stickstoffverbindung III durch den Rest R⁴(R³)CH- unter Freisetzung von einem Moläquivalent Wasser ersetzt.

Die Substituenten R¹ bis R¹⁰, die Variablen X, Y und die Indizes j, k, l, m, n und q in den Verbindungen I, III, VI und VII haben unabhängig voneinander folgende Bedeutungen:
R¹, R², R³, R⁴, R⁵, R⁶, R⁷, R⁸, R⁹, R¹⁰:
   - Wasserstoff (H), (R¹ und R² sind nicht beide gleichzeitig H),
R³, R⁴:
   - Alkyl, wie C₁₋₂₀-Alkyl, bevorzugt C₁₋₁₄-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl,
   - Hydroxyalkyl, wie C₁₋₂₀Hydroxyalkyl, bevorzugt C₁₋₈-Hydroxyalkyl, besonders bevorzugt C₁₋₄-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-(Hydroxymethyl)ethyl,
   - Aminoalkyl, wie C₁₋₂₀-Aminoalkyl, bevorzugt C₁₋₈-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(2-Aminoethyl)-2-aminoethyl und N-(2-Aminoethyl)aminomethyl,
   - Hydroxyalkylaminoalkyl, wie C₂₋₂₀₋Hydroxyalkylaminoalkyl, bevorzugt C₃₋₈-Hydroxyalkylaminoalkyl, wie (2-Hydroxyethylamino)methyl, 2-(2-Hydroxy-ethylamino)ethyl und 3-(2-Hydroxyethylamino)propyl,
   - R⁵-(OCR⁶R⁷CR⁸R⁹)ₙ-(OCR⁶R⁷), bevorzugt R⁵-(OCHR⁷CHR⁹)ₙ-(OCR⁶R⁷), besonders bevorzugt R⁵-(OCH₂CHR⁹)ₙ-(OCR⁶R⁷),
   - Alkylaminoalkyl, wie C₂₋₃₀-Alkylaminoalkyl, bevorzugt C₂₋₂₀-Alkylaminoalkyl, besonders bevorzugt C₂₋₈-Alkylaminoalkyl, wie Methylaminomethyl, 2-Methylaminoethyl, Ethylaminomethyl, 2-Ethylaminoethyl und 2-(iso-Propylamino)ethyl, (R⁵)HN-(CH₂)_{q},
   - Y-(CH₂)ₘ-NR⁵-(CH₂)_{q},
   - Heteroarylalkyl, wie C₄₋₂₀-Heteroarylalkyl, wie Pyrid-2-yl-methyl, Furan-2-yl-methyl, Pyrrol-3-yl-methyl und Imidazol-2-yl-methyl,
   - Alkylheteroaryl, wie C₄₋₂₀-Alkylheteroaryl, wie 2-Methyl-3-pyridinyl, 4,5-Dimethyl-imidazol-2-yl, 3-Methyl-2-furanyl und 5-Methyl-2-pyrazinyl,
   - Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,
R¹, R², R3, R⁴:
   - Cycloalkyl, wie C₃₋₁₂-Cycloalkyl, bevorzugt C₃₋₈-Cycloalkyl, wie Cyclopropyl, Cyclobutyl, Cyclopentyl, Cyclohexyl, Cycloheptyl und Cyclooctyl, besonders bevorzugt Cyclopentyl und Cyclohexyl,
   - Alkoxyalkyl, wie C₂₋₃₀-Alkoxyalkyl, bevorzugt C₂₋₂₀-Alkoxyalkyl, besonders bevorzugt C₂₋₈-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxyethyl, besonders bevorzugt C₂₋₄-Alkoxyalkyl,
   - Dialkylaminoalkyl, wie C₃₋₃₀-Dialkylaminoalkyl, bevorzugt C₃₋₂₀-Dialkylaminoalkyl, besonders bevorzugt C₃₋₁₀-Dialkylaminoalkyl, wie N,N-Dimethylaminomethyl, (N,N-Dibutylamino)methyl, 2-(N,N-Dimethylamino)ethyl, 2-(N,N-Diethyl-amino)ethyl, 2-(N,N-Dibutylamino)ethyl, 2-(N,N-Di-n-propylamino)ethyl und 2-(N,N-Di-iso-propylamino)ethyl, 3-(N,N-Dimethylamino)propyl, (R⁵)₂N-(CH₂)_{q},
   - Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
   - Alkylaryl, wie C₇₋₂₀-Alkylaryl, bevorzugt C₇₋₁₂-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
   - Aralkyl, wie C₇₋₂₀-Aralkyl, bevorzugt C₇₋₁₂-Phenylalkyl, wie Benzyl, p-Methoxybenzyl, 3,4-Dimethoxybenzyl, 1-Phenethyl, 2-Phenethyl, 1-Phenyl-propyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
   - R³ und R⁴ oder R² und R⁴ gemeinsam eine -(CH₂)ₗ-X-(CH₂)ₘ- Gruppe, wie-((CH₂)₃-, -(CH₂)₄-, -(CH₂)₅-, -(CH₂)₆-, -(CH₂)₇-, -(CH₂)-O-(CH₂)₂-, -(CH₂)-NR⁵-(CH₂)₂-, -(CH₂)-CHR⁵-(CH₂)₂-, -(CH₂)₂-O-(CH₂)₂-, -(CH₂)₂-NR⁵-(CH₂)₂-, -(CH₂)₂-CHR⁵-(CH₂)₂-, -CH₂-O-(CH₂)₃-, -CH₂-NR⁵-(CH₂)₃-, -CH₂-CHR⁵-(CH₂)₃-,
R¹, R²_{:}
   - Alkyl, wie C₁₋₂₀-Alkyl, bevorzugt C₁₋₈-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, 2-Ethylhexyl, besonders bevorzugt C₁₋₄-Alkyl, oder
   - R¹ und R² gemeinsam eine -(CH₂)ⱼ-X-(CH₂)ₖ- Gruppe, wie -(CH₂)₃-, -(CH₂)₄-,-(CH₂)₅-, -(CH₂)₆-, -(CH₂)₇-, -(CH₂)-O-(CH₂)₂-, -(CH₂)-NR⁵-(CH₂)₂-, -(CH₂)-CHR⁵-(CH₂)₂-, -(CH₂)₂-O-(CH₂)₂-, -(CH₂)₂-NR⁵-(CH₂)₂-, -(CH₂)₂-CHR⁵-(CH₂)₂-, -CH₂-O-(CH₂)₃-, -CH₂-NR⁵-(CH₂)₃-, -CH₂-CHR⁵-(CH₂)₃-,
R⁵, R¹⁰:
   - Alkyl, bevorzugt C₁₋₄-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
   - Alkylphenyl, bevorzugt C₇₋₄₀-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl, insbesondere C₇₋₂₀-Alkylphenyl,
R⁶, R⁷, R⁸, R⁹:
   - Methyl oder Ethyl, bevorzugt Methyl,
X:
   - CH₂, CHR⁵, Sauerstoff (O), Schwefel (S) oder NR⁵, bevorzugt CH₂ und O,
Y:
   - N(R¹⁰)₂, bevorzugt NH₂ und N(CH₃)₂,
   - Hydroxy (OH),
   - C₂₋₂₀-Alkylaminoalkyl, bevorzugt C₂₋₁₆-Alkylaminoalkyl, wie Methylaminomethyl, 2-Methylaminoethyl, Ethylaminomethyl, 2-Ethylaminoethyl und 2-(iso-Propylamino)ethyl,
   - C₃₋₂₀-Dialkylaminoalkyl, bevorzugt C₃₋₁₆-Dialkylaminoalkyl, wie Dimethylaminomethyl, 2-Dimethylaminoethyl, 2-Diethylaminoethyl, 2-(Di-n-propylamino)ethyl und 2-(Di-iso-propylamino)ethyl,
j, l:
   - eine ganze Zahl von 1 bis 4 (1, 2, 3 oder 4), bevorzugt 2 und 3, besonders bevorzugt 2,
k, m, q:
   - eine ganze Zahl von 1 bis 4 (1, 2, 3 oder 4), bevorzugt 2, 3 und 4, besonders bevorzugt 2 und 3,
n:
   - eine ganze Zahl von 1 bis 30, bevorzugt eine ganze Zahl von 1 bis 8 (1, 2, 3, 4, 5, 6, 7 oder 8), besonders bevorzugt eine ganze Zahl von 1 bis 6.

Als im erfindungsgemäßen Verfahren einsetzbare Ketone eignen sich unter den o.g. Voraussetzungen praktisch alle aliphatischen und aromatischen Ketone. Die aliphatischen Ketone können geradkettig, verzweigt oder zyklisch sein, die Ketone können Heteroatome enthalten. Die Ketone können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Ketone aminiert werden, so hat man es über die Steuerung der Reaktionsbedingungen in der Hand, Aminoketone, Aminoalkohole, cyclische Amine oder mehrfach aminierte Produkte zu erhalten.

Bevorzugt werden beispielsweise die folgenden Ketone aminierend hydriert:

Aceton, Ethylmethylketon, Methylvinylketon, Isobutylmethylketon, Butanon, 3-Methylbutan-2-on, Diethylketon, Tetralon, Acetophenon, Propiophenon, p-Methylacetophenon, p-Methoxy-acetophenon, m-Methoxy-acetophenon, 1-Acetyl-naphthalin, 2-Acetyl-naphthalin, 1-Phenyl-3-butanon, Cyclobutanon, Cyclopentanon, Cyclopentenon, Cyclohexanon, Cyclohexenon, 2,6-Dimethylcyclohexanon, Cycloheptanon, Cyclododecanon, Acetylaceton, Methylglyoxal und Benzophenon.

Als im erfindungsgemäßen Verfahren einsetzbare Aldehyde eignen sich unter den o.g. Voraussetzungen praktisch alle aliphatischen und aromatischen Aldehyde. Die aliphatischen Aldehyde können geradkettig, verzweigt oder zyklisch sein, die Aldehyde können Heteroatome enthalten. Die Aldehyde können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Aldehyde oder Ketoaldehyde aminiert werden, so hat man es über die Steuerung der Reaktionsbedingungen in der Hand, Aminoalkohole, cyclische Amine oder mehrfach aminierte Produkte zu erhalten.

Bevorzugt werden beispielsweise die folgenden Aldehyde aminierend hydriert:

Formaldehyd, Acetaldehyd, Propionaldehyd, n-Butyraldehyd, Isobutyraldehyd, Pivalinaldehyd, n-Pentanal, n-Hexanal, 2-Ethylhexanal, 2-Methylpentanal, 3-Methylpentanal, 4-Methylpentanal, Glyoxal, Benzaldehyd, p-Methoxybenzaldehyd, p-Methylbenzaldehyd, Phenylacetaldehyd, (p-Methoxy-phenyl)acetaldehyd, (3,4-Dimethoxyphenyl)-acetaldehyd, 4-Formyltetrahydropyran, 3-Formyltetrahydrofuran, 5-Formylvaleronitril, Citronellal, Lysmeral, Acrolein, Methacrolein, Ethylacrolein, Citral, Crotonaldehyd, 3-Methoxypropionaldehyd, 3-Aminopropionaldehyd, Hydroxypivalinaldehyd, Dimethylolpropionaldehyd, Dimethylolbutyraldehyd, Furfural, Glyoxal, Glutaraldehyd sowie hydroformylierte Oligomere und Polymere, wie z.B. hydroformyliertes Polyisobuten (Polyisobutenaldehyd) oder durch Metathese von 1-Penten und Cyclopenten erhaltenes und hydroformyliertes Oligomer.

Als Aminierungsmittel bei der hydrierenden Aminierung von Aldehyden und/oder Ketonen in Gegenwart von Wasserstoff können primäre oder sekundäre, aliphatische oder cycloaliphatische oder aromatische Amine eingesetzt werden.

Aus Di- oder Oligoaldehyden bzw. Di- oder Oligoketonen bzw. Ketoaldehyden lassen sich durch intramolekulare hydrierende Aminierung zyklische Amine, wie z.B. Pyrrolidine, Piperidine, Hexamethylenimine, Piperazine und Morpholine, herstellen.

Bevorzugt werden die primären oder sekundären Amine als Aminierungsmittel zur Herstellung unsymmetrisch substituierter Di- oder Trialkylamine, wie Ethyldiisopropylamin und Ethyldicyclohexylamin verwendet.

Beispielsweise werden die folgenden Mono- und Dialkylamine als Aminierungsmittel verwendet: Methylamin, Dimethylamin, Ethylamin, Diethylamin, n-Propylamin, Di-n-propyl-amin, iso-Propylamin, Di-isopropyl-amin, Dimethylmorpholin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin.

Mit dem erfindungsgemäßen Verfahren besonders bevorzugt hergestellte Amine sind zum Beispiel N,N-Di(C₁₋₄-alkyl)cyclohexylamin (aus Cyclohexanon und Di(C₁₋₄-alkyl)-amin), n-Propylamine (wie Dimethylpropylamin) (aus Propionaldehyd und DMA), N,N-Dimethyl-N-isopropylamin (aus Aceton und DMA), N,N-Dimethyl-N-butylamine (aus Butanal, i-Butanal oder Butanon und DMA), N-Ethyl-N,N-diisopropylamin (aus Acetaldehyd und N,N-Diisopropylamin), cis-4-[3-(4-tert-butylphenyl)-2-methylpropyl]-2,6-dimethylmorpholin (aus Lysmeral und cis-2,6-Dimethylmorpholin) und Tris(2-ethylhexyl)-amin (aus 2-Ethylhexanal und Di(2-ethylhexyl)amin).

### Beispiele

### Herstellung eines erfindungsgemäßen Katalysators

Al₂O₃-Stränge (2,8 mm Durchmesser) mit einer Oberfläche von 60-90 m²/g wurden mit einer Tränklösung, enthaltend Palladiumnitrat, Palladiumnitrit und Silbernitrat, welche mit konzentrierter (69 %iger) Salpetersäure auf einen pH-Wert im Bereich von 0,2 bis 2 angesäuert wurde, behandelt. Der Gehalt der zugesetzten Salpetersäure betrug in der fertigen Tränklösung 1,8 Gew.-%. Die feuchten Stränge wurden bei 200°C getrocknet und bei 600°C kalziniert. Es wurde ein Katalysator erhalten, der 0,3 Gew.-% Palladium und 0,1 Gew.-% Silber enthielt, wobei das Gewichtsverhältnis von Palladium zu Silber 3 betrug. Die Verteilung der Elemente über den Strangdurchschnitt gemessen mit der Electron Probe Micro Analysis (EPMA) Technik war wie folgt:
Mindestens 80 % des Palladiums (Pd) lag in einer Schicht zwischen der Oberfläche des Katalysators und einer Eindringtiefe, die maximal 80 % des Radius des Katalysators, gerechnet von der Oberfläche des Katalysators, entsprach, vor und der Promotor (Ag) lag über den gesamten Querschnitt des Katalysators vor.

Die folgenden Versuche erfolgten in einem elektrisch beheizten 1-Liter Rohrreaktor mit kontinuierlicher Reaktionsführung. Die Reaktionsausträge wurden mittels Gaschromatographie analysiert. Als Messprogramme wurden eingesetzt: a) Trennsäule DB1, Länge 60 m; Innendurchmesser 0,32 mm; Trägergas Helium; Temperaturprogramm: 80°C, danach mit 8°C/Minute auf 280°C, abschließend 15 Minuten isotherm bei 280°C und b) Trennsäule Rtx-5-Amine, Länge 30 m; Innendurchmesser 0,32 mm; Trägergas Helium; Temperaturprogramm: 70°C für 5 Minuten, danach mit 5°C/Minute auf 280°C, abschließend 10 Minuten isotherm bei 280°C. Die Angabe der Produktzusammensetzung erfolgt als GC-Flächenprozente der Rohausträge, wasserfrei und ohne den Überschuss an Einsatzstoff-Aminkomponente gerechnet.

### Beispiel 1: Fenpropimorph

Fenpropimorph (cis-FPM) wurde durch reduktive Aminierung aus Lysmeral-technisch (Lyal) und cis-2,6-Dimethylmorpholin (DMM) in Gegenwart von Wasserstoff und eines erfindungsgemäßen Festbettkatalysators hergestellt. Die Reaktion wurde in der Flüssigphase (Sumpf- oder Rieselfahrweise) durchgeführt.

In den Reaktor wurden die Edukte Lysmeral-technisch (ca. 95 %ig) und Dimethylmorpholin (> 97 %ig) mit getrennten Zuläufen bei 50 bis 140 bar Gesamtdruck eindosiert und bei 200 bis 240 °C im geraden Durchgang mit Flüssigkeitsrückführung umgesetzt. Die Synthese erfolgte mit einer Katalysatorbelastung von 0,25 bis zu 0,50 kg (Lyal)/(Liter(Kat.)•h) bei einem molaren DMM/Lyal-Verhältnis von 2,5. Die Einsatzstoffe Lysmeral und Dimethylmorpholin wurden nahezu quantitativ umgesetzt. Die Reaktion verlief sehr selektiv, weswegen nur geringe Mengen an Nebenkomponenten im Reaktionsaustrag vorlagen. Nebenkomponenten der Reaktion waren Lysmerol (Lyol) aus der unselektiven Hydrierung von Lysmeral, Lyol ist zusätzlich zu 2 % im Einsatzmaterial enthalten, und die Enamine aus Lysmeral und DMM (Zwischenstufe in der Synthese von cis-FPM). Die Versuchsergebnisse sind in folgender Tabelle 1 aufgelistet.

**Tabelle 1: Synthese von Fenpropimorph (Sumpf- und Rieselfahrweise)**

| Laufzeit [h] | Temperatur [°C] | Druck [bar] | Belastung [kg/Liter/h] | cis-FPM [%] | Lyol [%] | Lyal [%] | Enamine [%] |
|---|---|---|---|---|---|---|---|
| 0-200 | 200 | 90 | 0,31 | 97 | 2 | 0,1 | 0,5 |
| 200-350 | 220 | 90 | 0,31 | 97 | 2 | 0,1 | 0,5 |
| 350-500 | 220 | 140 | 0,31 | 97 | 2 | 0,1 | 0,5 |
| 500-700 | 200 | 140 | 0,31 | 97 | 2 | 0,1 | 1 |
| 700-800 | 200 | 50 | 0,31 | 97 | 2 | 0,2 | 1 |
| 800-1000 | 220 | 50 | 0,31 | 97 | 2 | 0,1 | 0,5 |
| 1000-1200* | 200 | 90 | 0,31 | 91 | 2 | 1 | 6 |
| 1200-1300* | 220 | 90 | 0,31 | 94 | 2 | 0,5 | 3 |
| 1300-1400* | 220 | 140 | 0,31 | 93 | 3 | 0,5 | 3 |
| 1400-1500* | 220 | 50 | 0,31 | 93 | 2 | 0,5 | 4 |
| 2200-2300 | 240 | 90 | 0,5 | 96 | 3 | 0,1 | 1 |
| 2300-2400 | 240 | 140 | 0,5 | 96 | 3 | 0,1 | 1 |
| 2400-2500 | 240 | 50 | 0,5 | 97 | 2 | 0,1 | 1 |
| 2500-2600 | 220 | 140 | 0,25 | 96 | 3 | 0,1 | 1 |
| 2800-2900 | 240 | 90 | 0,25 | 96 | 3 | 0,1 | 0,5 |
| 2900-3000 | 240 | 50 | 0,25 | 96 | 3 | 0,1 | 0,5 |
| 3000-3100 | 220 | 90 | 0,25 | 96 | 3 | 0,1 | 1 |

| | | | | | | | |
|---|---|---|---|---|---|---|---|
| *) in Rieselfahrweise, restliche Einträge: Sumpffahrweise | | | | | | | |

### Vergleichsbeispiel 1: Fenpropimorph

Fenpropimorph wurde durch reduktive Aminierung aus Lysmeral-technisch und cis-2,6-Dimethylmorpholin im gleichen Reaktor wie in Beispiel 1 hergestellt. Als Katalysator wurde ein silber- und palladiumhaltiger Festbettkatalysator eingesetzt, der Siliciumdioxid als Träger aufwies und keine Schalenstruktur der katalytisch aktiven Metalle besaß. Die Versuche wurden ansonsten unter vergleichbaren Reaktionsbedingungen durchgeführt. Aufgrund einer vergleichsweise geringen Katalysatoraktivität wurden oftmals erhöhte Enamin-Gehalte im Reaktionsaustrag ermittelt. Die Reaktion verlief weniger selektiv, vermehrt wurde als Nebenkomponente Lysmerol gebildet. Die Versuchsergebnisse sind in folgender Tabelle 2 zusammengefasst.

**Tabelle 2: Synthese von Fenpropimorph (Sumpffahrweise)**

| Laufzeit [h] | Temperatur [°C] | Druck [bar] | Belastung [kg/Liter/h] | cis-FPM [%] | Lyol [%] | Lyal [%] | Enamine [%] |
|---|---|---|---|---|---|---|---|
| 150-250 | 190 | 50 | 0,31 | 89 | 3 | 1 | 7 |
| 250-350 | 170 | 50 | 0,31 | 82 | 3 | 2 | 13 |
| 500-580 | 170 | 140 | 0,31 | 87 | 4 | 1 | 8 |
| 580-620 | 190 | 140 | 0,31 | 91 | 4 | 1 | 4 |
| 620-660 | 240 | 140 | 0,31 | 95 | 4 | 0,1 | 0,5 |

### Beispiel 2: Dimethylcyclohexylamin

N,N-Dimethylcyclohexylamin (DMCHA) wird durch reduktive Aminierung aus Cyclohexanon (Anon) und Dimethylamin (DMA) in Gegenwart von Wasserstoff und eines erfindungsgemäßen Festbettkatalysators hergestellt. Die Reaktion wird in der Flüssigphase in Riesel- oder Sumpffahrweise durchgeführt.

Die Edukte Cyclohexanon (99,5 %ig) und Dimethylamin (> 99 %ig) wurden bei 90 bis 130 bar Gesamtdruck in den Reaktor eindosiert. Die getrennten Zuläufe wurden vor dem Reaktor gemischt. Die Umsetzung erfolgte bei 160 bis 220°C im geraden Durchgang ohne Flüssigkeitsrückführung. Die Synthese erfolgte mit einer Katalysatorbelastung von 0,15 bis zu 0,80 kg (Anon)/(Liter(Kat.)•h) bei einem molaren DMA/Anon-Verhältnis von 2,3 bis 3,0. Die Einsatzstoffe Cyclohexanon und Dimethylamin wurden nahezu quantitativ umgesetzt. Die Reaktion verlief sehr selektiv, weswegen nur geringe Mengen an Alkohol (Cyclohexanol) im Reaktionsaustrag vorlagen. Eine andere Nebenreaktion war das "Scrambling", die Bildung von Monomethylcyclohexylamin (MMCHA) durch Methylgruppenwanderung beim DMCHA und DMA. Die Versuchsergebnisse sind in folgender Tabelle 3 aufgelistet.

**Tabelle 3: Synthese von DMCHA (Sumpffahrweise)**

| Laufzeit [h] | Temperatur [°C] | Druck [bar] | Molverhältnis Amin [Keton] | Belastung [kg/L/h] | DMCHA [%] | MMCHA [%] | Cyclohexanol [%] | Cyclohexanon [%] |
|---|---|---|---|---|---|---|---|---|
| 100-250 | 160 | 130 | 2,3 | 0,15 | 97 | 0,1 | 1 | 2 |
| 250-350 | 190 | 130 | 2,3 | 0,3 | 98 | 0,5 | 1 | 0,5 |
| 350-400 | 220 | 130 | 2,3 | 0,45 | 96 | 3 | 1 | 0,1 |
| 400-700 | 195 | 90 | 2,3 | 0,45 | 98 | 1 | 0,5 | 0,5 |
| 700-800 | 220 | 130 | 2,3 | 0,8 | 96 | 3 | 0,5 | 0,5 |
| 800-1000 | 205 | 130 | 3,0 | 0,6 | 97 | 2 | 0,5 | 0,1 |

### Vergleichsbeispiel 2: Dimethylcyclohexylamin

N,N-Dimethylcyclohexylamin wurde durch reduktive Aminierung von Cyclohexanon und Dimethylamin im gleichen Reaktor wie in Beispiel 2 hergestellt. Als Katalysator wurde ein silber- und palladiumhaltiger Festbettkatalysator eingesetzt, der Siliciumdioxid als Träger aufwies und keine Schalenstruktur der katalytisch aktiven Metalle besaß. Die Versuche wurden ansonsten unter vergleichbaren Reaktionsbedingungen durchgeführt. Die Reaktion verlief weniger selektiv, vermehrt wurde als Nebenkomponente Cyclohexanol durch Hydrierung von Cyclohexanon gebildet. Die Versuchsergebnisse sind in Tabelle 4 zusammengefasst.

**Tabelle 4: Synthese von DMCHA (Sumpffahrweise)**

| Laufzeit [h] | Temperatur [°C] | Druck [bar] | Molverhältnis Amin [Keton] | Belastung [kg/Liter/h] | DMCHA [%] | MMCHA [%] | Cyclohexanol [%] | Cyclohexanon [%] |
|---|---|---|---|---|---|---|---|---|
| 0-100 | 160 | 130 | 2,2 | 0,2 | 94 | 0,2 | 5 | 0,5 |
| 100-150 | 160 | 130 | 2,2 | 0,3 | 96 | 0,1 | 3 | 1 |
| 150-250 | 160 | 100 | 2,2 | 0,3 | 97 | 0,1 | 2 | 1 |
| 250-300 | 160 | 85 | 2,2 | 0,3 | 96 | 0,1 | 2 | 1,5 |
| 300-350 | 170 | 85 | 2,2 | 0,3 | 96 | 0,1 | 2 | 1,5 |
| 350-400 | 180 | 85 | 2,2 | 0,3 | 96 | 0,5 | 2 | 1 |

## Patentansprüche

1. Verfahren zur Herstellung eines Amins durch Umsetzung eines Aldehyds und/oder Ketons mit Wasserstoff und einer Stickstoffverbindung, ausgewählt aus der Gruppe primärer und sekundärer Amine, in Gegenwart eines Heterogenkatalysators, **dadurch gekennzeichnet, dass** es sich bei dem Katalysator um einen Schalenkatalysator handelt, der mindestens ein Metall der VIII. Gruppe des Periodensystems der Elemente als Hydriermetall und zusätzlich einen Promotor auf einem oxidischen Träger umfasst, wobei mindestens 80 % des Metalls der VIII. Gruppe des Periodensystems der Elemente in einer Schicht zwischen der Oberfläche des Katalysators und einer Eindringtiefe, die maximal 80 % des Radius des Katalysators, gerechnet von der Oberfläche des Katalysators, entspricht, vorliegt, und der Promotor über den gesamten Querschnitt des Katalysators im Wesentlichen homogen verteilt ist.

2. Verfahren nach Anspruch 1, **dadurch gekennzeichnet, dass** der eingesetzte Katalysator einen Durchmesser von 1,5 bis 10 mm aufweist und mindestens 80 % des Metalls der VIII. Gruppe des Periodensystems der Elemente in einer Schicht zwischen der Oberfläche des Katalysators und einer Eindringtiefe von maximal 1000 µm, gerechnet von der Oberfläche des Katalysators, vorliegt.

3. Verfahren nach den Ansprüchen 1 oder 2, **dadurch gekennzeichnet, dass** der oxidische Träger des Katalysators Aluminiumoxid ist.

4. Verfahren nach einem der vorhergehenden Ansprüche, **dadurch gekennzeichnet, dass** der oxidische Träger des eingesetzten Katalysators Aluminiumoxid in einer Mischung aus δ-, θ- und α-Aluminiumoxid ist.

5. Verfahren nach einem der vorhergehenden Ansprüche, **dadurch gekennzeichnet, dass** beim Katalysator das Metall der VIII. Gruppe des Periodensystems der Elemente Palladium ist.

6. Verfahren nach einem der vorhergehenden Ansprüche, **dadurch gekennzeichnet, dass** der Katalysator einen Gehalt an Metall der VIII. Gruppe des Periodensystems von 0,05 bis 5 Gew.-%, bezogen auf das Gesamtgewicht des Katalysators, aufweist.

7. Verfahren nach einem der vorhergehenden Ansprüche, **dadurch gekennzeichnet, dass** der Promotor des Katalysators ein Metall der IB. Gruppe des Periodensystems der Elemente ist.

8. Verfahren nach Anspruch 7, **dadurch gekennzeichnet, dass** das Metall der IB. Gruppe des Periodensystems der Elemente Silber ist.

9. Verfahren nach Anspruch 7 oder 8, **dadurch gekennzeichnet, dass** das Atomverhältnis zwischen dem Metall der VIII. Gruppe des Periodensystems der Elemente zum Metall der IB. Gruppe des Periodensystems der Elemente 0,1 bis 10 beträgt.

10. Verfahren nach einem der vorhergehenden Ansprüche, **dadurch gekennzeichnet, dass** der Katalysator hergestellt wird, indem ein oxidischer Träger mit einer Lösung, die Nitrat- und Nitritsalze von Metallen der VIII. und IB. Gruppe des Periodensystems der Elemente enthält und mit Salpetersäure angesäuert ist, getränkt, getrocknet und kalziniert wird.

11. Verfahren nach einem der vorhergehenden Ansprüche, **dadurch gekennzeichnet, dass** der Katalysator in einem Reaktor als Festbett angeordnet ist.

12. Verfahren nach einem der vorhergehenden Ansprüche, **dadurch gekennzeichnet, dass** die Umsetzung kontinuierlich stattfindet.

13. Verfahren nach einem der vorhergehenden Ansprüche, **dadurch gekennzeichnet, dass** die Umsetzung in der Flüssigphase durchgeführt wird.

14. Verfahren nach einem der vorhergehenden Ansprüche, **dadurch gekennzeichnet, dass** die Umsetzung in der Flüssigphase oder in einer gemischten Flüssig-/Gasphase mit mindestens 50 Gew.-% des Reaktionsgemisches in der Flüssigphase durchgeführt wird.

15. Verfahren nach einem der vorhergehenden Ansprüche, **dadurch gekennzeichnet, dass** die Umsetzung bei einer Katalysatorbelastung - gemessen als Masse an Aldehyd oder Keton im Zulauf bezogen auf das Katalysatorvolumen und die Zeit - im Bereich von 0,01 bis 2,00 kg (Carbonylverbindung)/Liter(Katalysator)/h durchgeführt wird.

16. Verfahren nach einem der vorhergehenden Ansprüche, **dadurch gekennzeichnet, dass** die Umsetzung bei einer Temperatur im Bereich von 50 bis 280°C durchgeführt wird.

17. Verfahren nach einem der vorhergehenden Ansprüche, **dadurch gekennzeichnet, dass** die Umsetzung bei einem Absolutdruck im Bereich von 1 bis 325 bar durchgeführt wird.

18. Verfahren nach einem der vorhergehenden Ansprüche, **dadurch gekennzeichnet, dass** die Stickstoffverbindung in der 0,90- bis 100-fachen molaren Menge bezogen auf den/das eingesetzte/n Aldehyd und/oder Keton eingesetzt wird.

19. Verfahren nach einem der Ansprüche 1 bis 18 zur Herstellung von cis-4-[3-(4-tert.-Butylphenyl)-2-methylpropyl]-2,6-dimethylmorpholin durch Umsetzung von Lysmeral mit cis-2,6-Dimethylmorpholin.

20. Verfahren nach einem der Ansprüche 1 bis 18 zur Herstellung von N,N-Dimethylcyclohexylamin durch Umsetzung von Cyclohexanon mit Dimethylamin.

21. Verfahren nach einem der Ansprüche 1 bis 18 zur Herstellung von N,N-Dimethyl-N-isopropylamin durch Umsetzung von Aceton mit Dimethylamin.

22. Verfahren nach einem der Ansprüche 1 bis 18 zur Herstellung von N-Ethyl-N,N-diisopropylamin (Hünig-Base) durch Umsetzung von Acetaldehyd mit N,N-Diisopropylamin.

23. Verfahren nach einem der Ansprüche 1 bis 18 zur Herstellung von Dicyclohexylamin durch Umsetzung von Cyclohexanon mit Cyclohexylamin.

24. Verfahren nach einem der Ansprüche 1 bis 18 zur Herstellung von Butylethylamin durch Umsetzung von Butanal mit Ethylamin.

25. Verfahren nach einem der Ansprüche 1 bis 18 zur Herstellung von Tris(2-ethylhexyl)amin durch Umsetzung von Ethylhexanal mit Bis(2-ethylhexyl)amin.

26. Verfahren nach einem der Ansprüche 1 bis 18 zur Herstellung von N-(Cyclododecyl)-2,6-dimethylmorpholin durch Umsetzung von Cyclododecanon mit trans-2,6-Dimethylmorpholin.

## Claims

1. A process for preparing an amine by reacting an aldehyde and/or ketone with hydrogen and a nitrogen compound selected from the group of primary and secondary amines in the presence of a heterogeneous catalyst, wherein the catalyst is a coated catalyst which comprises at least one metal of group VIII of the Periodic Table of the Elements as a hydrogenating metal and additionally a promoter on an oxidic support, at least 80% of the metal of group VIII of the Periodic Table of the Elements being present in a layer between the surface of the catalyst and a penetration depth which is not more than 80% of the radius of the catalyst, calculated from the surface of the catalyst, and the promoter being distributed essentially homogeneously over the entire cross section of the catalyst.

2. The process according to claim 1, wherein the catalyst used has a diameter of from 1.5 to 10 mm and at least 80% of the metal of group VIII of the Periodic Table of the Elements is present in a layer between the surface of the catalyst and a penetration depth of not more than 1000 µm, calculated from the surface of the catalyst.

3. The process according to claims 1 and 2, wherein the oxidic support of the catalyst is alumina.

4. The process according to any of the preceding claims, wherein the oxidic support of the catalyst used is alumina in a mixture of δ-, θ- and α-alumina.

5. The process according to any of the preceding claims, wherein the metal of group VIII of the Periodic Table of the Elements in the catalyst is palladium.

6. The process according to any of the preceding claims, wherein the catalyst has a content of metal of group VIII of the Periodic Table of from 0.05 to 5% by weight, based on the total weight of the catalyst.

7. The process according to any of the preceding claims, wherein the promoter of the catalyst is a metal of group IB of the Periodic Table of the Elements.

8. The process according to claim 7, wherein the metal of group IB of the Periodic Table of the Elements is silver.

9. The process according to claim 7 or 8, wherein the atomic ratio between the metal of group VIII of the Periodic Table of the Elements to the metal of group IB of the Periodic Table of the Elements is from 0.1 to 10.

10. The process according to any of the preceding claims, wherein the catalyst is prepared by impregnating an oxidic support with a solution which comprises nitrate and nitrite salts of metals of group VIII and IB of the Periodic Table of the Elements and has been acidified with nitric acid, drying it and calcining it.

11. The process according to any of the preceding claims, wherein the catalyst is arranged as a fixed bed in a reactor.

12. The process according to any of the preceding claims, wherein the reaction takes place continuously.

13. The process according to any of the preceding claims, wherein the reaction is performed in the liquid phase.

14. The process according to any of the preceding claims, wherein the reaction is performed in the liquid phase or in a mixed liquid/gas phase with at least 50% by weight of the reaction mixture in the liquid phase.

15. The process according to any of the preceding claims, wherein the reaction is performed at a catalyst hourly space velocity - measured as a mass of aldehyde or ketone in the feed based on the catalyst volume and time - in the range from 0.01 to 2.00 kg (carbonyl compound)/liter(catalyst)/h.

16. The process according to any of the preceding claims, wherein the reaction is performed at a temperature in the range from 50 to 280°C.

17. The process according to any of the preceding claims, wherein the reaction is performed at an absolute pressure in the range from 1 to 325 bar.

18. The process according to any of the preceding claims, wherein the nitrogen compound is used in from 0.90 to 100 times the molar amount based on the aldehyde and/or ketone used.

19. The process according to any of claims 1 to 18 for preparing cis-4-[3-(4-tert-butylphenyl)-2-methylpropyl]-2,6-dimethylmorpholine by reacting lysmeral with cis-2,6-dimethylmorpholine.

20. The process according to any of claims 1 to 18 for preparing N,N-dimethylcyclohexylamine by reacting cyclohexanone with dimethylamine.

21. The process according to any of claims 1 to 18 for preparing N,N-dimethyl-N-isopropylamine by reacting acetone with dimethylamine.

22. The process according to any of claims 1 to 18 for preparing N-ethyl-N,N-diisopropylamine (Hünig's base) by reacting acetaldehyde with N,N-diisopropylamine.

23. The process according to any of claims 1 to 18 for preparing dicyclohexylamine by reacting cyclohexanone with cyclohexylamine.

24. The process according to any of claims 1 to 18 for preparing butylethylamine by reacting butanal with ethylamine.

25. The process according to any of claims 1 to 18 for preparing tris(2-ethylhexyl)amine by reacting ethylhexanal with bis(2-ethylhexyl)amine.

26. The process according to any of claims 1 to 18 for preparing N-(cyclododecyl)-2,6-dimethylmorpholine by reacting cyclododecanone with trans-2,6-dimethylmorpholine.

## Revendications

1. Procédé de fabrication d'une amine par mise en réaction d'un aldéhyde et/ou d'une cétone avec de l'hydrogène et un composé d'azote choisi dans le groupe des amines primaires et secondaires, en présence d'un catalyseur hétérogène, **caractérisé en ce que** le catalyseur est un catalyseur à enveloppe qui comprend au moins un métal du groupe VIII du tableau périodique des éléments en tant que métal d'hydrogénation et également un promoteur sur un support oxydique, au moins 80 % du métal du groupe VIII du tableau périodique des éléments se trouvant dans une couche entre la surface du catalyseur et une profondeur de pénétration qui correspond au plus à 80 % du rayon du catalyseur, calculée à partir de la surface du catalyseur, et le promoteur étant réparti de manière essentiellement homogène sur l'ensemble de la section du catalyseur.

2. Procédé selon la revendication 1, **caractérisé en ce que** le catalyseur utilisé présente un diamètre de 1,5 à 10 mm et au moins 80 % du métal du groupe VIII du tableau périodique des éléments se trouve dans une couche entre la surface du catalyseur et une profondeur de pénétration d'au plus 1 000 µm, calculée à partir de la surface du catalyseur.

3. Procédé selon la revendication 1 ou 2, **caractérisé en ce que** le support oxydique du catalyseur est de l'oxyde d'aluminium.

4. Procédé selon l'une quelconque des revendications précédentes, **caractérisé en ce que** le support oxydique du catalyseur utilisé est de l'oxyde d'aluminium en un mélange d'oxyde d'aluminium δ, θ et α.

5. Procédé selon l'une quelconque des revendications précédentes, **caractérisé en ce que** le métal du groupe VIII du tableau périodique des éléments du catalyseur est le palladium.

6. Procédé selon l'une quelconque des revendications précédentes, **caractérisé en ce que** le catalyseur présente une teneur en métal du groupe VIII du tableau périodique des éléments de 0,05 à 5 % en poids, par rapport au poids total du catalyseur.

7. Procédé selon l'une quelconque des revendications précédentes, **caractérisé en ce que** le promoteur du catalyseur est un métal du groupe IB du tableau périodique des éléments.

8. Procédé selon la revendication 7, **caractérisé en ce que** le métal du groupe IB du tableau périodique des éléments est l'argent.

9. Procédé selon la revendication 7 ou 8, **caractérisé en ce que** le rapport atomique entre le métal du groupe VIII du tableau périodique des éléments et le métal du groupe IB du tableau périodique des éléments est de 0,1 à 10.

10. Procédé selon l'une quelconque des revendications précédentes, **caractérisé en ce que** le catalyseur est fabriqué par imprégnation d'un support oxydique avec une solution qui contient des sels de nitrate et de nitrite de métaux du groupe VIII et IB du tableau périodique des éléments et qui est acidifiée avec de l'acide nitrique, séchage et calcination.

11. Procédé selon l'une quelconque des revendications précédentes, **caractérisé en ce que** le catalyseur est agencé dans un réacteur sous la forme d'un lit fixe.

12. Procédé selon l'une quelconque des revendications précédentes, **caractérisé en ce que** la réaction a lieu en continu.

13. Procédé selon l'une quelconque des revendications précédentes, **caractérisé en ce que** la réaction est réalisée en phase liquide.

14. Procédé selon l'une quelconque des revendications précédentes, **caractérisé en ce que** la réaction est réalisée en phase liquide ou dans une phase liquide/gaz mixte avec au moins 50 % en poids du mélange réactionnel dans la phase liquide.

15. Procédé selon l'une quelconque des revendications précédentes, **caractérisé en ce que** la réaction est réalisée à une charge du catalyseur, mesurée en tant que masse d'aldéhyde ou de cétone dans l'alimentation par rapport au volume du catalyseur et au temps, dans la plage allant de 0,01 à 2,00 kg (composé de carbonyle)/litre (catalyseur)/h.

16. Procédé selon l'une quelconque des revendications précédentes, **caractérisé en ce que** la réaction est réalisée à une température dans la plage allant de 50 à 280 °C.

17. Procédé selon l'une quelconque des revendications précédentes, **caractérisé en ce que** la réaction est réalisée à une pression absolue dans la plage allant de 1 à 325 bar.

18. Procédé selon l'une quelconque des revendications précédentes, **caractérisé en ce que** le composé d'azote est utilisé en une quantité molaire de 0,90 à 100 fois l'aldéhyde et/ou la cétone utilisés.

19. Procédé selon l'une quelconque des revendications 1 à 18, pour la fabrication de cis-4-[3-(4-tert.-butylphényl)-2-méthylpropyl]-2,6-diméthylmorpholine par mise en réaction de Lysmeral avec de la cis-2,6-diméthylmorpholine.

20. Procédé selon l'une quelconque des revendications 1 à 18, pour la fabrication de N,N-diméthylcyclohexylamine par mise en réaction de cyclohexanone avec de la diméthylamine.

21. Procédé selon l'une quelconque des revendications 1 à 18, pour la fabrication de N,N-diméthyl-N-isopropylamine par mise en réaction d'acétone avec de la diméthylamine.

22. Procédé selon l'une quelconque des revendications 1 à 18, pour la fabrication de N-éthyl-N,N-diisopropylamine (base d'Hünig) par mise en réaction d'acétaldéhyde avec de la N,N-diisopropylamine.

23. Procédé selon l'une quelconque des revendications 1 à 18, pour la fabrication de dicyclohexylamine par mise en réaction de cyclohexanone avec de la cyclohexylamine.

24. Procédé selon l'une quelconque des revendications 1 à 18, pour la fabrication de butyléthylamine par mise en réaction de butanal avec de l'éthylamine.

25. Procédé selon l'une quelconque des revendications 1 à 18, pour la fabrication de tris(2-éthylhexyl)amine par mise en réaction d'éthylhexanal avec de la bis(2-éthylhexyl)amine.

26. Procédé selon l'une quelconque des revendications 1 à 18, pour la fabrication de N-(cyclododécyl)-2,6-diméthylmorpholine par mise en réaction de cyclododécanone avec de la trans-2,6-diméthylmorpholine.
